(19) Europäisches Patentamt

European Patent Office

Office européen des brevets

(11) EP 0 969 018 A1

(12) **EUROPEAN PATENT APPLICATION**

(43) Date of publication:
**05.01.2000 Bulletin 2000/01**

(51) Int Cl.⁷: **C07K 16/12**, G01N 33/577, C12N 5/20, C07K 14/245

(21) Application number: **98305260.6**

(22) Date of filing: **02.07.1998**

(84) Designated Contracting States:
**AT BE CH CY DE DK ES FI FR GB GR IE IT LI LU MC NL PT SE**
Designated Extension States:
**AL LT LV MK RO SI**

(71) Applicant: **Combact Diagnostic Systems Ltd.**
**Herzliya 46766 (IL)**

(72) Inventor: **Burowski, Daphna**
**Tel-Aviv 69419 (IL)**

(74) Representative: **Marchant, James Ian et al**
**Elkington and Fife,**
**Prospect House,**
**8 Pembroke Road**
**Sevenoaks, Kent TN13 1XR (GB)**

(54) **Anti bacterial monoclonal antibodies**

(57) Hybridoma cell lines are described which produce MAbs capable of binding to a high extent to specific bacterial cells as compared to their low extent of binding or no binding to other kinds of bacterial cells. The described MAbs are mostly such capable of binding to a high extent to bacterial cells which it is comparatively easy to grow in culture.

**Description**

**FIELD OF THE INVENTION**

The present invention concerns hybridoma cell lines which produce anti-bacterial antibodies, antibodies produced by the cell lines and various uses thereof.

**GLOSSARY**

The following terms will be used throughout the description below:
**Infectious bacteria** - bacteria which are capable of causing infection in an individual. Infectious bacteria may be bacteria which are not normally present and once present give rise to an infection. Infectious bacteria may also be a bacteria which are normally present, but at a low concentration, an infection is then a result of increase in their concentration. Such bacteria may also form part of the normal body flora in their native form but as a result of genetic mutation may become infectious. In clinical samples, the presence of infectious bacteria above a certain critical level is considered a sign of infection.
**Non-specific bacterial monoclonal antibodies (MAbs) .** MAbs which are capable of binding to antigens expressed by bacterial cells belonging to more than one type of bacterial strain.
**Specific anti-bacterial MAbs -** MAbs which bind to antigens expressed by bacterial cells of a specific bacterial group (i.e. family, genus, species or strain) at a much higher extent than to antigens expressed by bacterial cells belonging to other bacterial groups.
**Antibody sensitivity -** a measure of antibody's capability to identify samples containing cells belonging to a strain, species, genus or family, to which the antibodies are specific (specific cells). This is typically measured by determining the percent of test samples containing the specific cells to which specific antibodies bind. An antibody will be considered to be a sensitive antibody if it is capable to bind to at least 90% of test samples containing the specific cells.
**Antibody specificity -** a measure of the ability of antibodies to distinguish between samples containing specific and such containing other cells (non specific cells). This is typically measured by determining the percent of tested samples containing non specific cells to which the antibody bind. An antibody will be considered to have a high specificity if, on the one hand, it has a high sensitivity, and on the other hand, binds to no more than 10% of samples which contain non specific cells and which do not contain specific cells.

**BACKGROUND OF THE INVENTION**

The determination of the presence of infectious bacteria in a tested sample and the identification of the type of the specific type of infectious bacteria in the sample is of great importance in tests carried out in many fields including medical diagnostic tests, water and food quality control tests, air pollution tests, etc. In the medical field, clinical diagnosis of tested.body fluid samples are of particular importance since the correct identification of the type of infectious bacteria in the sample may have a crucial effect on the correct diagnosis, treatment regimen and prognosis of the infected individual. Such accurate identification of the infecting bacteria is also necessary for epidimiology purposes and may also be of particular importance in the evaluation of infected water, food, cosmetic and pharmaceutical samples wherein the origin of infection must be identified and correctly treated.
Most of the assays used to date for identification of infectious bacteria in a sample require to culture and sub-culture the tested sample in order to grow individual isolated colonies present in the sample. In most cases physiological and biochemical properties of the individual isolated colonies are then determined by biochemical assays known in the art, such as for example, fermentation of certain sugars, ability to grow on selective media, etc.
The abovementioned common methods used to date for identification of infectious bacteria in a sample require a long period of time for obtaining results (being an average of about two days) as well as to carry out a large series of tests (usually between 10 and 30 tests per sample).
Methods which require a substantially shorter period of time for obtaining results and involve only a single test are also available. Such tests are based on the detection of specific antigens expressed by the bacteria present in the sample. For this purpose, it is necessary to use antibodies which are capable of binding to an antigen which is expressed only by bacterial cells belonging to a specific type of bacterial group, and not by bacterial cells belonging to a different bacterial group type, i.e. bacterial specific MAbs. Such MAbs should also have the capability to bind to all bacterial cells belonging to the bacterial group which expresses the antigen recognized by them, i.e. such having high sensitivity.
A number of methods and systems for the detection of infectious bacteria in a tested sample and for the identification of the specific type of bacteria present in an infected tested sample are known which utilize specific anti-bacterial antibodies. Most of these methods involve use of known enzyme linked immunoassays (ELISA) or different agglutination assays. One such method is described, for example, in Hayashi-K *et al.,* (Kansenshogaku-Zasshi, **65**: 457-464, 1991), wherein an ELISA kit is used for the detection of Chlamydia trachomatis antigen. Another example for such a method is described in Farrington, M., *et al.*, *J. Clin. Pathol.,* **44**:670-675, 1991, wherein a reverse passive hemagglutination microtiter based assay was used for streptococcal grouping.

The above methods are used today mainly for the detection of bacteria of the kind which it is either difficult or impossible to grow in culture or for such bacteria which grow inside eukaryotic cells (e.g. M. tuberculosis, *Chlamydia Spp,* etc.).

MAbs capable of binding antigens expressed by bacterial cells belonging to bacterial groups such as *E.coli, Klebsiella spp, Enterobacter spp.* which are capable of growing in culture relatively easily, have also been described and are available commercially. However, many of the available anti-bacterial monoclonal antibodies are not sufficiently sensitive and/or not sufficiently specific in order to give highly reliable identification results. Some antibodies are not capable of detecting the presence of infectious bacterial cells in a sample in which the concentration of the bacteria is under a certain critical concentration. Other antibodies are highly cross-reactive, i.e. bind to antigens present on cells belonging to several types of bacterial strains or on non-bacterial components present at times in the tested sample (such as cell debris) and as a result, correct classification of the infectious bacteria in the tested sample is not possible. Due to the low sensitivity or specificity of such antibodies, methods utilizing them may give rise to a large number of false positive or false negative results.

**GENERAL DESCRIPTION OF THE INVENTION**

In accordance with the present invention, it has become possible to detect and identify infectious bacteria of the kind which grows relatively easy in culture by a direct method which is substantially faster than the abovementioned available methods (2-3 hours as compared to about two days). The method of the invention which is based on utilization of highly sensitive and highly specific monoclonal antibodies also requires only a single test for identification of the infectious bacteria.

It is the main object of the present invention to provide hybridoma cell lines which produce monoclonal antibodies capable of binding to a high extent to specific bacterial cells while binding to a low extent or not at all to non-specific bacterial cells; i.e. specific anti-bacterial monoclonal antibodies having a high sensitivity and a high specificity.

In accordance with one aspect of the present invention, there are thus provided mouse hybridoma cell lines which produce specific anti-bacterial monoclonal antibodies having high sensitivity and specificity, Specific embodiments of such hybridoma cell lines are such which produce monoclonal antibodies (MAbs) which are sensitive and specific for *Enterobacteriaceae E. coli, Klebsiella spp, Pseudomonas spp.* and *Proteus Spp.*

Specific examples of hybridoma cell lines in accordance with the invention are the following:

1. A hybridoma cell line designated herein as *"anti-E.coli"* hybridoma which was deposited on 18.6.97 in the Centre for Applied Microbiology and Research (CAMR), European Collection of Cell Cultures (ECACC) depository under Accession No. 97061801.

2. A hybridoma cell line designated herein as *"anti-Klebsiella"* hybridoma which was deposited on 12.2.97 in the CAMR/ECACC depository under Accession No. 97021226.

3. A hybridoma cell line designated herein as *"anti-Pseudomonas"* which was deposited on 18.6.97 in the CAMR/ECACC depository under Accession No. 97061802.

4. A hybridoma cell line designated herein as *"anti-Proteus"* hybridoma which was deposited on 18.6.97 in the CAMR/ECACC depository under Accession No. 97061803.

5. A hybridoma cell line designated herein as *"anti-Enterobacteriaceae"* which was deposited on 12.2.97 in the CAMR/ECACC depository under Accession No. 97021227.

For the preparation of hybridomas, in accordance with the invention, hosts; e.g. mice are immunized with cells of a bacterial strain and after receiving several recurrent immunizations, their spleens are removed. Cell suspensions are prepared from the spleens and the antibody producing cells obtained from the immunized mice are fused with a fusion partner, e.g. a mouse fusion partner, an example being the non secreting myeloma cell line NSO/1 by one of the fusion methods known in the art (e.g. Kohler G. and Milstein C., *Nature,* **256**:495-497, 1975). The supernatants of the hybridoma cell line are typically screened for antibody binding activity by any one of the methods known in the art such as by enzyme linked immunosorbent assay (ELISA) or radioimmuno assay (RIA). The supernatant of Hybridomas producing antibacterial MAbs. are screened for production of antibacterial antibodies by their capability to bind to specific bacterial cells, using one of the abovementioned methods. In order to isolate the antibodies produced by the selected hybridoma cells lines in accordance with the invention, preferably, the hybridoma cell lines are cultured in vitro in a suitable medium wherein the desired monoclonal antibody is recovered from the supernatant. In some cases, MAbs secreted by the antibacterial hybridomas may also be obtained by injecting the hybridoma cell lines intraperitoneally (i.p.) into mice and then harvesting the antibodies secreted by the hybridomas from the malignant ascitic fluid formed in the injected mice or from the serum of the injected mice. For various purposes the supernatent of the selected hybridomas may also be used without further isolation of the monoclonal antibodies from the supernatant.

By an additional aspect of the present invention, MAbs produced by any one of the abovementioned hybridoma cell lines are provided. Such MAbs are selected from the group consisting of:

(i) anti-bacterial MAbs, e.g. *anti-E.coli, anti-Enterobacteriaceae, anti-Proteus, anti-Klebsiella* or *anti-Pseudomonas-specific* MAbs;
(ii) fragments of the antibodies of (i) retaining the antigen binding characteristics of the whole antibody; and
(iii) antibodies capable of binding to the antigenic epitope bound by any one of the antibodies of (i) and (ii) above.

Monoclonal antibodies secreted by the *anti-E.coli* specific hybridoma cell line will be designated herein as *"anti-E.coli-specific Mab".,* antibodies secreted by the *anti-Enterobacteriaceae* specific hybridoma will be designated as *"anti-Enterobacteriaceae* specific Mab", antibodies secreted by the *anti-Proteus* cell line will be designated as *"anti-proteus specific MAb",* antibodies secreted by the *anti-Klebsiella* cell line will be designated as *"anti-Klebsiella-specific* MAb" and the antibodies produced by the anti-Pseudonomas hybridoma cell line will be designated as *"anti-Pseudonomas-specific Mab".*

In accordance with a diagnostic aspect of the invention, compositions comprising the antibodies of the invention may be used for diagnosis to detect the presence of bacteria in a tested sample and to identify the type of infectious bacteria in the sample. The invention therefore provides, by one of its further aspects, a diagnostic reagent composition comprising MAbs belonging to at least one of the abovementioned antibodies together with a suitable carrier. The carrier may either be a soluble carrier such as any one of the physiologically acceptable buffers known in the art (e.g. PBS) or a solid phase carrier such as, for example, latex beads.

In accordance with the invention, for diagnostic purposes a body fluid specimen is first obtained from the individual to be tested for the preparation of infected bacteria. The specimen may either be tested directly or after having been cultured under conditions which enable growth and isolation of infectious bacteria present in the obtained specimen. Alternatively, the presence of infectious bacteria in the obtained sample may be first detected by using any of the methods known in the art such as a chemical non-specific staining assay capable of staining bacteria present in the sample. In yet other cases, symptoms typical of conditions caused by specific kinds of bacteria may be present in the individual from which the sample was obtained.

Samples prepared from the obtained specimen are reacted with the diagnostic reagent composition of the invention under conditions permitting the formation of antibody-antigen complexes. Such complexes may be formed by binding of the MAbs of the invention to bacterial cells expressing the specific antigen or, alternatively, by binding of the MAbs to isolated bacterial antigens. The extent of binding of the MAbs to the bacteria or antigens is then determined by methods known in the art, such as for example, by immuno-fluoresence assays, radio immunoassays (RIA) or enzyme linked immunosorbent assays (ELISA). A level of bound MAbs significantly higher than that observed in a control sample indicating the presence of specific infecting bacteria in the tested sample.

In accordance with the invention, a diagnostic reagent composition may either comprise a single kind of anti-bacterial specific antibody or, alternatively, several types of specific anti-bacterial antibodies, each labeled by a different distinguishable labei. The label may be, *a priori,* bound to the monoclonal antibody, or alternatively, the label may be bound to a carrier molecule which then specifically binds to the bacteria specific antibody. The carrier molecule may, for example, be an antibody which specifically binds to the bacteria specific antibody. Alternatively, the antibody may be conjugated to biotin and the carrier molecule may then be avidin. As will be appreciated by the artisan, the above two labeling embodiments are only examples and any other labeling technique which will allow to identify the binding of the antibacterial antibody to the specific bacterial cells may be used in accordance with the invention.

Where a diagnostic reagent composition comprising a single antibody is used, typically, a plurality of samples from the obtained specimen are separately and simultaneously incubated with antibody compositions, each comprising a different antibacterial specific monoclonal antibody. Where a composition comprising a plurality of monoclonal antibodies of the invention are used, each with different antibacterial specificity (and each associated, for example, with a fluorescent dye having or giving rise to a different color) the number of simultaneously incubated samples may be reduced.

In both above cases, the incubation of the tested sample with the diagnostic reagent composition is for a time sufficient to allow binding of the monoclonal antibodies to the bacterial cells. Following incubation and labeling, the microorganisms may go through several additional procedures (such as, for example, filtering through a filter) and may then be transferred for viewing in the microscope, the detection, for example, of a certain fluorescent color being an indication of the presence of a certain type of bacteria in the infected sample.

By a most preferred embodiment of the invention, compositions comprising antibodies of the invention are used in rapid diagnostic assays. One example for such a rapid diagnostic assay is the Bactis™ system (Combact Diagnostic Systems Ltd., Israel) described in IL Patent Application No. 104351. The high sensitivity and specificity of the antibodies of the, invention render them to be most suitable for such rapid tests in which any non specific binding may significantly distort the results.

Fragments of the antibodies of the invention which substantially maintain the antigen binding characteristics of the whole antibody are also within the scope of the present invention. Such fragments may be, for example, Fab or F(ab)2 fragments obtained by digestion of the whole antibody with various enzymes as known and extensively described in the art. The antigenic characteristics of an antibody or fragment thereof may be determined by testing the binding of the antibody to a certain antigenic determinant using any one of the known standard assays such as RIA, ELISA or FACS analysis.

By a further therapeutical aspect of the invention, pharmaceutical compositions comprising the monoclonal antibacterial specific antibodies of the invention may be used for the treatment of various bacterial infections in an individual by administering to such a patient a therapeutic effective amount of the monoclonal antibody or portion thereof capable of binding to the infectious bacteria. A therapeutically effective amount being an amount capable of alleviating the symptoms of the bacterial infection or reducing the number of bacterial cells in an individual. Such pharmaceutical compositions may also be used, for example, for prevention or passive immunization of individuals against primary or possible recurrent bacterial infections. For this purpose, it may be preferable to use pharmaceutical compositions comprising one or more Mabs of the invention which were humanized by any one of the methods known in the art.

The antigen to which the antibodies of the invention specifically bind also constitutes an aspect of the invention. Pharmaceutical compositions comprising the antigens of the invention, may be used for active immunization of an individual to obtain a specific immune response against the bacteria expressing the injected antigen. In addition, compositions comprising bacterial antigens in accordance with the invention, may also be used for the detection and identification of an infectious bacteria in a body sample obtained from tested individual. In such an individual, endogenic antibodies may have developed as a result of the bacterial infection. Therefore, by obtaining a body fluid sample from a tested individual, incubating it with the antigen of the invention under conditions enabling the formation of antigen-antibody complexes, detecting the level of the formed complexes and comparing it to the level of complexes in a control sample, it is possible to determine whether the individual is indeed infected by the specific bacteria. For this purpose, the body sample will typically be a serum sample in which it is possible to detect even low levels of endogenic antibodies.

The present invention further provides a kit for use in the diagnosis or therapy of various bacterial infections comprising one or more of the antibodies of the invention and/or one or more of the antigens of the invention and any further reagents required for detecting the binding of such antibodies or antigens to bacteria present in a tested sample.

The invention will now be illustrated by way of the following examples which should not be construed to be limiting.

**EXAMPLES**

**Materials and Methods**

The following abbreviations will be used in the description below:

**ATCC** - American Type Culture Collection
**Ig** - immunoglobulin
**BSA** - bovine serum albumin
**FITC** - fluorescein iso-thio-cyanate
**MAb** - monoclonal antibody
**PBS** - phosphate buffered saline
**RAMIg** - rabbit anti mouse immunoglobulin
**TSB** - tryptose soy broth
**OPD** - ortho-phenylene diamine
**HRP** - horse radish peroxidase

**1. Preparation of bacteria for immunization:**
Bacterial strains (ATCC/mutant strains/clinical isolates) were identified using conventional methods typically being the Vitek GNI card (bioMerieux, Vitek Inc., USA). In the following, this method will be referred to *as "the reference method"*.

For immunization, the bacterial cells were inoculated in TSB and grown for 16-18 hours at 37°C with agitation. The cells were collected and washed three times with saline (0.15M NaCl). The cells were subsequently re-suspended in saline to a concentration of approximately $10^9$/ml and heated to 100°C for 60 mins. Either a single bacterial strain or a mixture of several strains (as indicated in the table below) was used for each immunization.

Table 1

| Bacterial strains used for the different immunization protocols | | | | | | | |
|---|---|---|---|---|---|---|---|
| | Priming | 1st boost | 2nd boost | 3rd boost | 4th boost | boost (day 3) | boost (day 2) |
| **Escherichia coli** (rough mutants) | K12 B | K12 B | 576 470 2131 2513 653 | 576 470 2141 2513 653 | | 576 470 2141 2513 653 | 576 470 2141 2513 653 |
| **Klebsiella pneumonia** (K-mutants) | K26-1 | K26-1 | K26-1 | K26-1 201 | K26-1 201 | K26-1 201 | K26-1 201 |
| **Pseudomonas aeruginosa** (K-mutant) | 21536• | 21536• | 21536• | 21536• | | 21536• | 21536• |
| **Proteus mirabilis vulgaris penneri** | 1537*, 29906* 515*, 3549* 3261*, 3489* | 2876*, 127* 1411* 2502* 881*, 33519* | 5422* 5434* 2645* 13315* 671*, 1422* | | | 2464* 1858* 861*, 2509* 3329*, 689* | 2461*, 1861* 253329* 6 |

* Combact isolates - Combact collection

• ATCC strains

## 2. Immunization schedule

Mice (Balb/C female, 8 weeks of age) were immunized intraperitoncally, with $10^8$ bacterial cells (mixture prepared as above), emulsified in Incomplete Freund's Adjuvant at two-weeks intervals. Number of boosts varied according to the bacteria involved. Last boosts were given after 5-12 weeks of rest, on day 3 (intraperitoneally) and day 2 (intravenously) before fusion (day 0).

## 3. Preparation of hybridomas

Somatic cell fusion of splenocytes, derived from immunized mice and the murine (non-secreting) myeloma cell-line NSO/1, was carried out according to the method published by Kohler and Milstein (Kohler, G. and Milstein, C., 1975 *Nature,* **256**:495-497).

## 4. Selection of positively producing hybridomas using the ELISA assay

4.1 Supernatants derived from hybridoma cultures were tested for binding activity to bacterial cells suitably prepared to enable specific MAb binding. ELISA plates were coated with bacterial cells prepared as for immunization. A mixture consisting of 10 distinct bacterial strains/clinical isolates (total $10^8$ cells) was added to each well and the plates dried for 16-18 hours at 55°C. Binding activity of a specific MAb (present in culture supernatants) to bacteria was determined by using HRP-rabbit anti-mouse Ig and OPD as substrate.

4.2 Selection of specific hybridomas: The binding of MAb to a plate coated with bacteria to which the desired MAb is specific (a) was compared to the binding to a plate coated with a mixture of bacteria to which the desired MAb is non-specific (b). The level of (a) and (b) was compared to that of the blank (wells in the ELISA plate to which all reagents were added with the exception of the MAb). If (a) > (blank) x 5 and (b) = blanks, the hybridoma was selected as positive and specific.

4.3 Selection of hybridomas exhibiting high sensitivity: The binding of MAb to ELISA plates coated with a representing minimal panel of 12 discrete bacterial strains/clinical isolates, to which the desired MAb is specific, was determined. Hybridomas showing partial recognition capacity to the tested panel, were discarded.

**5. Characterization of the produced monoclonal antibodies (sensitivity and specificity)**

5.1 For identification of bacteria using the MAbs of the invention:
The bacterial strains were inoculated in TSB and grown with agitation until they reached midlog phase. The cell suspensions were then used as described in 5.2 and 5.3 below. The tested sample comprised of 60-80 correspondent bacterial isolates and a similar number of non-correspodent *("negative")* bacterial isolates for each of the tested MAbs.

5.2 Indirect staining of the samples with MAbs and FITC-RAMIg (two-step procedure);
Pretreated bacterial cells were incubated with either culture supernatant or purified MAb (diluted in PBS, 1% BSA), for 60 minutes at $37^\circ$C. The cells were subsequently washed twice with the above diluent and incubated with FITC-RAMIg (diluted in PBS, 1% BSA) for 45 minutes at $37^\circ$C. The cells were then washed and microscopically inspected.

5.3 Indirect staining with Biotinylated-MAb and FITC-Avidin ;
<u>Isolation and purification of MAb from cell-culture medium.</u>
Hybridoma cells were adapted to growth in serum-free medium containing mg/ml BSA. MAb preparations were isolated and purified from cell culture supernatants either by affinity chromatography (Protein A/protein G-Sepharose), gel filtration or ion-exchanger chromatography according to the isotypic nature of the MAb.
<u>Labeling of MAb with Biotin</u>
Conjugation of biotin to MAb was done by using of biotinyl $\varepsilon$ - aminocaproyl N-hydroxysuccinimide ester, according to a protocol published by Bayer and Wilchek (Bayer E.A., and Wilchek. M., *Methods in Molecular Biology,* Vol. 10: *Immunochemical Protocols,* Chapter 13; 137-142, ed: M. Manson, The Human Press Inc., Iotowa, N.J., 1992).

<u>Staining procedure</u>
Bacterial cells, appropriately treated, were incubated with Biotinylated MAb (diluted in PBS, 1% BSA), for 30 minutes at $37^\circ$C. FITC-Nutralite™ (Pierce Chemical Company) - Avidin was then added (diluted in PBS, 1% BSA) and the cells were subsequently incubated for additional 30 minutes at $37^\circ$C. The cells were then washed and microscopically inspected.

**6. Urine Specimens:**
Fresh urine specimens were obtained from Sheba Medical Center, Israel. Infected urines containing Gram negative rods at a threshold of $\geq 10^5$ CFU/ml (as detected by the screening process) were tested in the identification process using the Mabs of the invention. The identification of low-prevalence bacterial species such as *Pseudomonas spp.* and *Proteus spp.* was done on sterile urine specimens, spiked with corresponding and non-corresponding bacterial isolates and processed as the native urines were processed. The microorganisms present in each urine specimen (either native or spiked) were identified in parallel using the reference method.

**7. Calculation of the Sensitivity (SE) and Specificity (SP) of the tested MAbs:**
The sensitivity and the specificity of the Mabs was evaluated in comparison to the reference method (see above) and calculated as follows:

$$\text{Sensitivity (SE)} = \frac{\text{True Postive results}}{\text{(True Positive + False Negative) results}}$$

$$\text{Specificity (SP)} = \frac{\text{True Negative results}}{\text{(True Positive + False Negative) results}}$$

**8. Preliminary characterization of the antigens**

8.1 <u>Sensitivity to proteolysis:</u>

8.1.1 A Proteinase-K stock solution was prepared by dissolving Proteinase-K in 0.02M, Tris-HCI buffer

pH-7.8 (final concentration 0.8 mg/ml).

8.1.1 The digestion step was performed as follows:

a protein solution corresponding to 80 µg protein was added to 2 µl of 0.5M Tris-HCl buffer pH-7.8 and 2 µl of 0.25% SDS solution. The final volume was adjusted with DDW to be 45 µl.

The above mixture was heated at 850C for 5 minutes, cooled down to room temperature, spun for 30 seconds at maximum speed in an Eppendorf centrifuge and 5 µl of Proteinase-K stock solution was then added. The protein to Proteinase-K quantity ratio was 20:1. Digestion proceeded for 2 hours at room temperature (R.T.). Sensitivity to Proteinase-K digestion was determined by the disappearance of a specifically identified band in a Western Blot following the above digestion procedure.

8.2 <u>Characterization by immunoblotting</u>

Immunoblotting was carried out as described in: *Antibodies,* Harlow E. and Lane, D., (Eds.), Cold Spring Harbor Laboratory, pp. 479-510, 1988.

The determination of the molecular weight of the antigens was carried out by comparing the location of the bands obtained from the samples to pre-stained SDS-page standards (Biorad Laboratories).

**RESULTS**

**Determination of sensitivity and specificity parameters for the anti-bacterial MAbs**

The specificity and sensitivity of the monoclonal antibodies produced by the hybridomas were determined by incubation of the antibodies as described above with a large number of tested samples obtained from type strains from the ATCC and bacterial isolates derived from various sources (hospitals in Israel and abroad) isolated mainly from urine. The samples were infected either with the bacteria against which the Abs are specifically directed or with different types of other bacteria as specified in each case below (Tables 2-6).

As seen in the following results, the MAbs of the invention were shown to be useful in the detection and identification of infectious bacteria in urine samples. The specificity and sensitivity of the MAbs was tested directly on urine specimens as described above.

**Example 1**

Table 2:

| Identification of *Enterobacteriaceae* | | | | | | |
|---|---|---|---|---|---|---|
| **Clinical isolates** | | | | **Urine Specimens** (117 specimens tested ) | | |
| Bacterial groups tested | Total tested | FP[1] results | FN[2] results | Total detected * | FP results | FN results |
| *E. coli* | 25 | | | 77 | | 1 |
| *Klebsiella pneumonia* | 17 | | | | | |
| *Klebsiella oxytoca* | 6 | | | | | |
| *Klebsiella spp.* | - | | | 28 | | 1 |
| *Morganella morganii* | 17 | | | 3 | | |
| *Citrobacter spp.* | 20 | | | 9 | | |
| *Enterobacter spp.* | 26 | | 2 | 13 | | |
| *Providencia spp.* | 15 | | | 2 | | |
| *Serratia marcesans.* | 15 | | | 2 | | |

* Detected by the reference method (see above)

Table 2:   (continued)

| Identification of *Enterobacteriaceae* | | | | | | |
|---|---|---|---|---|---|---|
| **Clinical isolates** | | | | **Urine Specimens** (117 specimens tested ) | | |
| Bacterial groups tested | Total tested | FP[1] results | FN[2] results | Total detected * | FP results | FN results |
| *Salmonella spp.* | 2 | | | - | | |
| **Total (positives)\*\*** | **143** | | 2 | **134** | | 2 |
| **Proteus spp.** | 19 | | 19 | 26 | | 26 |
| *Acinetobacter spp.* | 27 | | | 8 | | |
| *Pseudomonas spp.* | 35 | | | 9 | | |
| **Total (negatives)** | **62** | | | **17** | | |
| **Sensitivity (%)\*\*\*** | **98.6** | | | **98.5** | | |
| **Specificity (%)** | **100** | | | **100** | | |

\* Detected by the reference method (see above)

\*\* Proteus spp. excluded- was not identified by the *Enterabacteriaceae spp* - specific MAbs

\*\*\* For the calculation of the % sensitivity and % specificity see above
[1] FP = False Positive
[2] FN = False Negative

## Example 2

*E.coli* **- Specific Mab:**

Table 3:

| Idendification of *E. coli* | | | | | | |
|---|---|---|---|---|---|---|
| **Clinical isolates** | | | | **Urine Specimens** (332 specimens tested ) | | |
| Bacterial groups tested | Total tested | FP results | FN results | Total detected * | FP results | FN results |
| *E. coli* | 80 | | | 186 | | 9 |
| **Total positives** | **80** | | | **186** | | **9** |
| *Proteus mirabilis* | 17 | | | 40 | 1 | |
| *Klebsiella pneumonia* | 32 | | | - | | |
| *Klebsiella oxytoca* | 6 | | | - | | |
| *Klebsiella spp.* | | | | 82 | 2 | |
| *Morganella morganii* | 9 | | | - | | |
| *Citrobacter spp.* | 14 | | | 13 | 0 | |
| *Enterobacter spp.* | 20 | | | 16 | 1 | |
| *Providencia spp.* | 9 | | | - | | |
| *Acinetobacter spp.* | 7 | | | 26 | 1 | |
| *Pseudomonas spp.* | 8 | | | 23 | 1 | |
| *Other Gram negative* | - | | | 33 | 0 | |
| **Total (negatives)** | **122** | | | **233** | **6** | |
| **Sensitivity (%)** | **100** | | | **95.1** | | |

\* Detected by the reference method

Table 3: (continued)

| Idendification of *E. coli* | | | | | | |
|---|---|---|---|---|---|---|
| **Clinical isolates** | | | | **Urine Specimens** (332 specimens tested ) | | |
| Bacterial groups tested | Total tested | FP results | FN results | Total detected * | FP results | FN results |
| **Specificity (%)** | **100** | | | **97.4** | | |

\* Detected by the reference method

**Example 3**

*Proteus spp - * **specific MAb:**

Table 4:

| Identification of *Proteus spp.* | | | | | | |
|---|---|---|---|---|---|---|
| **Clinical isolates** | | | | **Urine Specimens** ( 85 spiked specimens tested) | | |
| Bacterial groups tested | Total teste d | FP results | FN results | Total tested | FP results | FN results |
| *Proteus mirabilis* | 50 | | | 24 | 3 | |
| *Proteus vulgaris* | 11 | | | 16 | 1 | |
| *Proteus penneri* | 17 | | | 20 | 2 | |
| **Total (positives)** | **78** | | | **60** | **6** | |
| *E. coli* | 11 | | | 3 | | |
| *Klebsiella pneumonia* | 7 | | | 2 | | |
| *Klebsiella oxytoca* | 4 | | | - | | |
| *Morganella morganii* | 5 | | | 2 | | |
| *Citrobacter spp.* | 7 | 1 | | 3 | | |
| *Enterobacter spp.* | 8 | | | 1 | | |
| *Providencia spp.* | 4 | | | 1 | | |
| *Serratia rettgeri* | 1 | | | | | |
| *Acinetobacter spp.* | 10 | | | 1 | | |
| *Pseudomonas spp.* | 6 | | | 12 | | |
| **Total (negatives)** | **63** | **1** | | **25** | | |
| **Sensitivity (%)** | **100** | | | **90** | | |
| **Specificity (%)** | **98.4** | | | **100** | | |

**Example 4**

*Pseudomonas spp.* **specific MAb:**

Table 5:

| Identification of *Pseudomonas spp.* | | | | | | |
|---|---|---|---|---|---|---|
| **Clinical isolates** | | | | **Urine Specimens** ( 86 spiked specimens tested) | | |
| Bacterial groups tested | Total tested | FP results | FN results | Total tested | FP results | FN results |
| *P. aeruginosa* | 67 | | 1 | 45 | | 4 |
| *P. putida* | 18 | | | 15 | | 1 |
| *P. stuzeri* | 2 | | | | | |
| *P. facilis* | 1 | | | | | |
| *P. cepacia* | 2 | | | | | |
| *P. fluorescens* | 1 | | 1 | | | |
| *P. diminuta* | 4 | | 4 | | | |
| **Total (positives)** | **95** | | **6** | **60** | | **5** |
| *E. coli* | 13 | | | 2 | | |
| *Klebsiella pneumonia* | 11 | | | 2 | | |
| *Klebsiella oxytoca* | 5 | | | 1 | | |
| *Morganella morganii* | 7 | | | 2 | | |
| *Proteus spp.* | 12 | | | 12 | | |
| *Citrobacter spp.* | 8 | | | 2 | | |
| *Enterobacter spp.* | 9 | | | 2 | | |
| *Providencia spp.* | 3 | | | 1 | | |
| *Acinetobacter spp.* | 9 | | | 2 | | |
| **Total (negatives)** | **77** | | | **26** | | |
| **Sensitivity (%)*** | **98.5** | | | **91.6** | | |
| **Specificity (%)** | **100** | | | **100** | | |

* w/o *Pseudomonas diminuta & Pseudomonas Fluorescens*
*(Pseudomonas fluorescens has* low virulence, without clinical significance
*Pseudomonas diminuta is rarely encountered in clinical environment)*

**Example 5**

*klebsiella spp -* **Specific MAbs:**

Table 6:

| Identification of *Klebsiella spp.* | | | | | | |
|---|---|---|---|---|---|---|
| **Clinical isolates** | | | | **Urine Specimens** (332 specimens tested) | | |
| Bacterial groups tested | Total tested | FP results | FN results | Total detected * | FP results | FN results |
| *Klebsiella pneumonia* | 50 | | 6 | - | | |
| *Klebsiella oxytoca* | 10 | | | - | | |
| *Klebsiella spp.* | - | | | 82 | | 5 |

* Detected by the reference method.

Table 6: (continued)

| Identification of *Klebsiella spp.* | | | | | | |
|---|---|---|---|---|---|---|
| **Clinical isolates** | | | | **Urine Specimens** (332 specimens tested) | | |
| Bacterial groups tested | Total tested | FP results | FN results | Total detected * | FP results | FN results |
| **Total (positives)** | **60** | | **6** | **82** | | **5** |
| *E. coli* | 57 | | | 186 | 8 | |
| *Proteus mirabilis* | 17 | 2 | | - | | |
| *Proteus spp.* | - | - | | 40 | 2 | |
| *Morganella morganii* | 9 | 1 | | - | - | |
| *Citrobacter spp.* | 14 | 5 | | 13 | 5 | |
| *Enterobacter spp.* | 20 | 2 | | 16 | 1 | |
| *Providencia spp.* | 9 | 1 | | - | - | |
| *Acinetobacter spp.* | 7 | | | 26 | 4 | |
| *Pseudomonas spp.* | 8 | | | 23 | | |
| Other Gram negatives | - | | | 33 | 1 | |
| **Total (negatives)** | **141** | 11 | | **337** | **21** | |
| **Sensitivity (%)** | **90.0** | | | **93.9** | | |
| **Specificity (%)** | **92.2** | | | **93.7** | | |

* Detected by the reference method.

**Example 6**

**Preliminary Characterization of the antigens to which the MAbs of the invention bind:**

6.1 <u>*E.coli:*</u>
The antigen recognized by the *E.coli* - specific MAbs of the Invention was found to be susceptible to Proteinase-K digestion. This was evidenced by the disappearance of a specific band in a Western Blot after proteolytic digestion (for procedure see above). The molecular weight of the *E.coli* antigen prepared from sonicated *E.coli* bacteria was determined by Western Blot and was found to be between about 11,000 to about 14,000.

6.2 *Enterobacteriaceae* antigen:
The antigen identified by the *Enterobacteriaceae-specific* MAbs of the invention was found to be susceptible to Proteinase-K digestion (determined as described above). The molecular weight of this antigen was found to be similar to that of the above-mentioned *E.coli* antigen, i.e., in the range of about 11,000 - 14,000.

6.3 <u>*Klebsiella*</u> antigen:
The antigen identified by the *Klebsiella-specific* MAbs of the invention was found to be not susceptible to Proteinase-K cleavage (determined as described above). This antigen was found to be associated with bacterial lipopolysaccharide (LPS).

**Claims**

1. A hybridoma cell line producing monoclonal antibodies capable of binding to a high extent to specific bacterial cells, said antibodies capable of binding to a low extent or not at all to non-specific bacterial cells.

2. A hybridoma cell line in accordance with Claim 1, wherein said specific bacterial cells are of the kind which it is relatively easy to grow in culture.

3. A hybridoma cell line capable of producing monoclonal antibodies specific for *E.coli spp ("anti-E.coli hybridoma")* which was deposited on 18.6.97 in the Centre for Applied Microbiology and Research (CAMR), European Collection of Cell Cultures (ECACC) depository under Accession No. 97061801.

4. A hybridoma cell line capable of producing MAbs specific for *Klebsiella spp ("anti-Kiebslella hybridoma")* which was deposited on 12.2.97 in the CAMR/ECACC depository under Accession No. 97021226.

5. A hybridoma cell line capable of producing MAbs specific for *Pseudomonas spp ("anti-Pseudomonas")* which was deposited on 18.6.97 in the CAMR/ECACC depository under Accession No.97061802.

6. A hybridoma cell line capable of producing MAbs specific for *Proteus spp ("anti-Proteus")* hybridoma which was deposited on 18.6.97 in the CAMR/ECACC depository under Accession No. 97061803.

7. A hybridoma cell line capable of producing MAbs specific for *Enterobacteriaceae spp ("anti-Enterobacteriaceae")* which was deposited on 12.2.97 in the CAMR/ECACC depository under Accession No. 97021227.

8. Monoclonal antibodies produced by the hybridoma cell lines of any one of the preceding claims selected from the group consisting of:

   (i) *anti-E.coli-specific* MAbs, *anti-Enterobacteriaceae-specific* MAbs, *anti-Proteus-specific* MAbs, *anti-Klebsiella-specific* MAbs or *anti-Pseudomonas-specific* MAbs;
   (ii) fragments of the antibodies of (i) retaining the antigen binding characteristics of the whole antibody; and
   (iii) antibodies capable of binding to the antigenic epitope bound by any one of the antibodies of (i) and (ii) above.

9. A diagnostic reagent composition comprising at least one monoclonal antibody of Claim 8 or a fragment thereof together with a suitable carrier.

10. A diagnostic reagent composition according to Claim 9, wherein the anti-bacterial antibody is labeled.

11. An antigen capable of binding to any one of the antibodies of Claim 8.

12. A kit for use in the diagnosis or therapy of bacterial infections comprising at least one of the antibodies of claim 8.

13. A kit for use in the diagnosis or therapy of bacterial infections comprising at least one antigen in accordance with claim 11.

14. A method for the diagnosis of a bacterial infection in an individual comprising the steps of:

   (i) obtaining a fluid specimen from the individual to be tested;
   (ii) preparing a number of samples from said obtained specimen;
   (iii) reacting each of said samples with a diagnostic reagent composition in accordance with Claim 9, each of said compositions comprising a different anti-bacterial specific MAb, each of said MAbs labeled with a detectable label under conditions permitting the formation of antibody-antigen complexes; and
   (iv) detecting the extent of binding of each of said diagnostic reagent compositions to said specific bacteria, a level of binding significantly higher than that observed in a control sample indicating the presence of said specific infecting bacteria in the sample and indicating the existence of a bacterial infection in said tested individual.

15. A method according to Claim 14, wherein only a single sample is prepared in step (ii) said sample being reacted in step (iii) with a diagnostic reagent composition comprising more than one anti-bacterial specific MAb, each of said MAbs being labeled by a different detectable label.

EP 0 969 018 A1

<table>
<tr><td colspan="5"><strong>European Patent Office</strong>    <strong>EUROPEAN SEARCH REPORT</strong>    Application Number<br>EP 98 30 5260</td></tr>
<tr><td colspan="5" align="center"><strong>DOCUMENTS CONSIDERED TO BE RELEVANT</strong></td></tr>
<tr><td>Category</td><td colspan="2">Citation of document with indication, where appropriate,<br>of relevant passages</td><td>Relevant<br>to claim</td><td>CLASSIFICATION OF THE<br>APPLICATION (Int.Cl.6)</td></tr>
<tr><td>X</td><td colspan="2">US 5 354 661 A (DOYLE MICHAEL P ET AL) 11<br>October 1994<br>* column 3, line 16-62 *<br>* column 4, line 17-54 *<br>* column 7, line 32 - column 8, line 5 *<br>---</td><td>1-3,8-15</td><td>C07K16/12<br>G01N33/577<br>C12N5/20<br>C07K14/245</td></tr>
<tr><td>X</td><td colspan="2">BUROWSKI D. ET AL.: "Rapid identification<br>of gram-negative bacteria directly in<br>urine specimens using the Bactis 160U"<br>ABSTRACTS OF THE GENERAL MEETING OF THE<br>AMERICAN SOCIETY FOR MICROBIOLOGY,<br>vol. 98, no. 0, 17 - 21 May 1998, C-463,<br>page 208 XP002086697<br>* the whole document *<br>---</td><td>1-3,8-15</td><td></td></tr>
<tr><td>X</td><td colspan="2">WO 86 01806 A (TECHNOLOGY LICENCE CO LTD)<br>27 March 1986<br>* page 7, line 31 - page 9, line 25 *<br>* example 1 *<br>* page 21, line 30 - page 23, line 28 *<br>---</td><td>1-3,8-15</td><td></td></tr>
<tr><td>X</td><td colspan="2">US 5 747 293 A (DOUGAN GORDON ET AL) 5<br>May 1998<br>* column 1, line 3-8 *<br>* column 1, line 48-57 *<br>* column 2, line 24-55 *<br>* examples 3,4 *<br>---</td><td>1-3,8-15</td><td>TECHNICAL FIELDS<br>SEARCHED (Int.Cl.6)<br><br>C07K<br>G01N</td></tr>
<tr><td>X</td><td colspan="2">WO 98 05687 A (US ARMY ;VIRION SYSTEMS INC<br>(US)) 12 February 1998<br>* page 1, line 34 - page 2, line 26 *<br>* page 7, line 5 - page 8, line 16 *<br>-----</td><td>1-3,8-15</td><td></td></tr>
<tr><td colspan="5" align="center">The present search report has been drawn up for all claims</td></tr>
<tr><td colspan="2">Place of search<br>THE HAGUE</td><td colspan="2">Date of completion of the search<br>3 December 1998</td><td>Examiner<br>Covone, M</td></tr>
<tr><td colspan="2">CATEGORY OF CITED DOCUMENTS<br><br>X : particularly relevant if taken alone<br>Y : particularly relevant if combined with another<br>   document of the same category<br>A : technological background<br>O : non-written disclosure<br>P : intermediate document</td><td colspan="3">T : theory or principle underlying the invention<br>E : earlier patent document, but published on, or<br>   after the filing date<br>D : document cited in the application<br>L : document cited for other reasons<br>.................................................................<br>& : member of the same patent family, corresponding<br>   document</td></tr>
</table>

EPO FORM 1503 03.82 (P04C01)

14